Europäisches Patentamt

European Patent Office

Office européen des brevets

(11) Numéro de publication : **0 496 680 B1**

(12) **FASCICULE DE BREVET EUROPEEN**

(45) Date de publication du fascicule du brevet :
06.09.95 Bulletin 95/36

(51) Int. Cl.⁶ : **G01N 33/569,** C12Q 1/04,
C12Q 1/37, C12N 1/20

(21) Numéro de dépôt : **92420012.4**

(22) Date de dépôt : **10.01.92**

(54) **Procédé et milieu d'identification de bactéries du genre Listeria.**

(30) Priorité : **16.01.91 FR 9100650**

(43) Date de publication de la demande :
**29.07.92 Bulletin 92/31**

(45) Mention de la délivrance du brevet :
**06.09.95 Bulletin 95/36**

(84) Etats contractants désignés :
**BE CH DE ES GB IT LI LU NL PT**

(56) Documents cités :
**FOOD MICROBIOLOGY, vol. 7, no. 2, 01 février
1990, New York, NY (US); F. DEL CORRAL et
al., pp. 99-106/**

(56) Documents cités :
**JOURNAL OF APPLIED MICROBIOLOGY, vol.
32, no. 3, 01 septembre 1969, Washington, DC
(US); P.A. KRAMER et al., pp. 381-394/
ANNALES PHARMACEUTIQUES FRANCAISE,
vol. 48, no. 6, 01 décembre 1990, Paris (FR); A.
AGBAN et al., pp. 326-334/**

(73) Titulaire : **BIO MERIEUX, Société anonyme
F-69280 Marcy l'Etoile (FR)**

(72) Inventeur : **Monget, Daniel
Résidence du Moulin,
13 rue de Moulin du Buis
F-01150 Saint Sorlin en Bugey (FR)**

(74) Mandataire : **Guerre, Dominique et al
CABINET GERMAIN et MAUREAU
Le Britannia Tour C
20 Bd E. Deruelle
F-69392 Lyon Cédex 03 (FR)**

## Description

La présente invention a pour objet un procédé et un milieu d'identification bactériologique, permettant de différencier et/ou identifier les différentes espèces du genre Listeria. Plus précisément, l'invention a pour objet principal la différenciation de l'espèce monocytogenes, pathogène, des autres espèces non pathogènes du genre Listeria.

Le genre Listeria comprend aujourd'hui sept espèces, dont seule Listeria monocytogenes est pathogène pour l'homme. Des germes sont à l'origine d'épidémies sévères chez l'homme, à la suite de contaminations à l'origine alimentaires. Dans les aliments (lait et produits laitiers essentiellement), deux espèces de Listeria sont principalement isolées : Listeria innocua non pathogène et Listeria monocytogenes pathogène.

Ces deux espèces ont de nombreux caractères biochimiques communs, il est en conséquence difficile de les différencier Food Microbiology, 1990, 7, 99-106, décrit un mode d'identification des Listeria. Les tests actuellement utilisés sont les suivants.

1) Le test de la β-hémolyse : ce test repose sur la détermination d'une activité β-hémolytique liée à la production par la bactérie d'une substance qui provoque la lyse des hématies (listériolysine). Ce test est exercé sur gélose de sang de mouton ou de cheval, mais la réponse est souvent discrète. Ce test est donc peu fiable et s'il permet de différencier les deux espèces principalement rencontrées, à savoir L. monocytogenes (réponse positive) de L. innocua (réponse négative), il n'est pas spécifique de l'espèce pathogène.

2) Le CAMP-test : ce test est associé au test de la B-hémolyse précédemment décrit. Le test est réalisé sur gélose trypticase soja, contenant 5 % d'hématies lavées de mouton. La souche β-hémolytique de S. aureus CIP 5710 est ensemencée en une strie perpendiculaire aux stries formées par la culture de la souche de Listeria à tester. Le renforcement de l'hémolyse du staphylocoque au contact des deux zones indique une réaction positive. En pratique, le CAMP-test est une technique lourde à mettre en oeuvre, et, comme l'hémolyse, n'est pas toujours très fiable.

La présente invention a donc pour objet principal un mode de différenciation de l'espèce monocytogenes du genre Listeria, particulièrement discriminant, sensible, et relativement peu subjectif.

A titre secondaire, la présente invention a pour objet un système complet d'identification des différentes espèces du genre Listeria.

Après avoir soumis les sept espèces du genre Listeria à l'épreuve de plus de 500 tests biochimiques différents, se décomposant en :
- 142 tests enzymat iques (peptidases, osidases, phosphatases, estérases, etc... )
- 82 tests de fermentation
- 278 tests d'auxonogrammes

la Demanderesse a abouti à la découverte fondamentale selon laquelle toutes les espèces de Listeria possédaient une activité enzymatique glycine aminopeptidase, à l'exception de l'espèce pathogène L. monocytogenes.

A partir de cette découverte, la présente invention propose donc un procédé d'analyse bactériologique particulièrement discriminant pour les espèces du genre Listeria et consistant à mettre en contact, sous incubation, l'échantillon à analyser avec un milieu d'identification comprenant un substrat de la glycine aminopeptidase, chromogène ou fluorigène, et susceptible d'être hydrolysé en présence de l'enzyme précité.

Toujours à partir de la même découverte, l'invention propose un milieu d'identification bactériologique des espèces du genre Listeria, par exemple conditionné sous forme déshydratée, comprenant un substrat de la glycine aminopeptidase, chromogène ou fluorigène, et susceptible d'être hydrolysé au contact ou en présence de l'échantillon à analyser, pouvant contenir l'espèce L. monocytogenes.

Aux fins de caractérisation des espèces Listeria autres que monocytogenes, un tel milieu peut aussi comprendre un ou plusieurs substrats de transformation permettant de caractériser l'espèce présente dans l'échantillon à analyser. Ils sont de préférence choisis parmi les substrats suivants :
- substrats fermentescibles par les bactéries, c'est-à-dire hydrates de carbone (D-xylose, ribose, rhamnose, tagatose, α-méthyl-D-mannoside, α-méthyl-D-glucoside, mannitol ...)
- substrats réductibles par les bactéries tels que nitrates, et de préférence le nitrate de potassium
- substrats hydrolysables par voie enzymatique tels que substrats de l'α-mannosidase.

Par substrat chromogène ou fluorigène, on entend tout marqueur dont la molécule, en présence d'au moins un enzyme spécifique, est susceptible d'être hydrolysée ou coupée en deux parties, à savoir une partie inerte, non chromogène ou fluorigène, et une partie chromogène ou fluorigène, pouvant développer une couleur visible à l'oeil nu, ou sous éclairement UV, directement, ou indirectement, c'est-à-dire par action d'un composé chimique complémentaire, dit révélateur.

Ainsi le substrat chromogène ou fluorigène peut être choisi parmi les substrats hydrolysables en un produit :

- directement fluorigène, à savoir la glycine-7-amido-4-méthylcoumarine, la glycine-7-amido-4-trifluorométhylcoumarine
- directement chromogène, à savoir le glycine-4-nitroanilide, et
- indirectement chromogène, à savoir le glycine-2-naphtylamide et le glycine-4-méthoxy-2-naphtylamide, auxquels est additionné un révélateur choisi parmi les sels de diazonium du type Fast Blue BB, et le paradiméthylaminocinnamaldéhyde.

On obtient ainsi un complexe coloré en présence d'une réaction positive. Bien entendu, les substrats ci-dessus ne sont donnés qu'à titre d'exemple, et ne constituent pas une limitation à la présente invention.

La concentration du substrat de la glycine aminopeptidase introduit dans le milieu, varie de préférence entre 0,01 mM et 10 mM.

Le milieu selon l'invention comprend un système tampon choisi parmi les tampons Tris, phosphatés et milieux peptonés. Il permet d'ajuster le pH du milieu à une valeur comprise entre 6 et 9.

Selon une formulation particulière du milieu de l'invention, le milieu contient du chlorure de sodium, pour une teneur de préférence comprise entre 0 et 10 g. Il peut également contenir au moins un activateur de la glycine aminopeptidase, en quantité préférentiellement comprise entre 0 et 100 mg pour 1 litre de milieu et choisi de préférence parmi les cations bivalents $Mg^{2+}$, $Ca^{2+}$, $Mn^{2+}$,... Selon cette formulation particulière, le milieu peut contenir une solution de $MnCl_2$ ou $MnSO_4$, de préférence à raison de 30 mg/l de milieu.

La préparation du milieu selon l'invention, la mise en oeuvre du procédé et les avantages sont à présent illustrés par les exemples suivants.

EXEMPLE 1 - Préparation d'un milieu selon l'invention

Une formulation préférentielle du milieu est la suivante :
- glycine-2-naphtylamide     0,65 g
- trizma base     6,06 g
- NaCl     5,00 g
- eau distillée     1l
- HCl     qsp pH 8

La formulation précédente est déshydratée dans des puits de plaque de microtitration par séchage ventilé à 37°C pendant 24 heures. Le volume de milieu à évaporer est de 100 microlitres par puits. Les milieux ainsi préparés sont conservés sous déshydratant, à l'obscurité à +4°C jusqu'à leur utilisation.

EXEMPLE 2 - Mise en oeuvre du procédé selon l'invention

La mise en oeuvre ci-dessous est donnée à titre d'exemple. Il est évident qu'elle peut subir de nombreuses variantes.

A partir d'un isolement sur gélose Columbia au sang de mouton (5 %), on réalise une suspension de la souche de Listeria à étudier, dans de l'eau distillée stérile. Cette suspension est ajustée au point N° 4 de l'échelle de McFarland. Cent microlitres de cette suspension sont introduits dans un puits de la plaque de microtitration contenant la formulation déshydratée décrite selon Exemple 1. Après une incubation de 4 heures à 37°C, une goutte de réactif Fast Blue BB est ajoutée au milieu réactionnel pour détecter la présence de 2-naphtylamine libre. Le développement d'une coloration orange indique une réaction positive.

EXEMPLE 3 - Utilisation du milieu selon l'invention pour différencier les deux espèces Listeria, L. monocytogenes et L. innocua

La mise en oeuvre du procédé selon Exemple 2, est appliquée à la différenciation des souches suivantes :
- 41 souches de L. monocytogenes
- 33 souches de L. innocua.
Les résultats obtenus ont été les suivants, exprimés en nombre de souches positives :
- L. monocytogenes : 0/41, soit 0 %
- L. innocua : 33/33, soit 100 %
Cet exemple met en évidence la spécificité du milieu et sa fiabilité résultant d'une observation qui ne prête à aucune confusion.

EXEMPLE 4 - Utilisation d'un milieu selon l'invention en combinaison avec d'autres milieux de détection pour identifier les espèces du genre Listeria

Les milieux de détection choisis, associés au milieu selon l'invention, qui ne sont qu'une illustration des possibilités offertes, sont les milieux suivants :
- milieu de détection des bactéries qui fermentent le D-xylose
- milieu de détection des bactéries qui fermentent le ribose
- milieu de détection des bactéries qui fermentent l'α-méthyl-D-mannoside
- milieu de détection des bactéries qui réduisent les nitrates.

La formulation de chacun des milieux est la suivante :
- milieu selon l'invention soit A, formulation selon Exemple 1
- milieu comprenant l'hydrate de carbone fermentescible, soit B
  . eau distillé          1000 ml
  . hydrate de carbone : D-xylose, ribose, α-Me-D-mannoside          10 g
  . bacto-peptone Difco          5 g
  . rouge de phénol          60 mg
  . pH ajusté à          8,5
- milieu comprenant les nitrates, soit C
  . eau distillée          1000 ml
  . nitrate de potassium          1 g
  . bacto-peptone Difco          10 g
  . pH ajusté à          7,0

Les différentes formulations ont été stérilisées par filtration sur filtre 0,22 micron, et réparties dans des puits de plaque de microtitration aux côtés du test glycine aminopeptidase précédemment décrit, à raison de 100 microlitres par puits. Les milieux réactionnels ont ensuite été déshydratés par séchage ventilé à 37°C pendant 24 heures.

Les batteries de tests ainsi préparées ont été conservées sous déshydratant à l'obscurité et à +4°C jusqu'à leur utilisation.

104 souches de Listeria ont été étudiées, réparties ainsi :
- 41 L. monocytogenes
- 33 L. innocua
- 8 L. ivanovii
- 3 L. welshimeri
- 14 L. seeligeri
- 3 L. grayi
- 2 L. murrayi.

Ces différentes souches ont été testées de la façon suivante :
- pour chaque souche isolée sur gélose Columbia au sang de mouton (5 %), une suspension bactérienne a été réalisée dans de l'eau distillée stérile
- cette suspension a été ajustée au point n° 4 de l'échelle de McFarland
- 100 microlitres ont été répartis dans les puits correspondant aux 5 milieux décrits ci-dessus
- après une incubation de 4 heures à 37°C, les différentes réactions ont été interprétées de la façon suivante :
  . milieu A : (voir Exemple 3)
  . milieu B :
       coloration jaune : réaction positive
       coloration rouge : réaction négative
  . milieu C : après addition d'une goutte de chacun des réactifs NIT1 et NIT2, commercialisés par BIO MERIEUX et consistant en une solution dans de l'acide acétique, respectivement d'acide sulfanilique et de N,N-diméthyl-1-naphtylamine
       coloration rouge : réaction positive
       pas de coloration : réaction négative

Les résultats obtenus ont été les suivants :

| Milieu / Espèce | Substrat | A | B | | | C |
|---|---|---|---|---|---|---|
| | | Substrat de la glycine aminopeptidase | D-xylose | Ribose | alpha-méthyl-D-mannoside | nitrate |
| L. monocytogenes | | − | − | − | + | − |
| L. innocua | | + | − | − | + | − |
| L. ivanovii | | + | + | + | − | − |
| L. welshimeri | | + | + | − | + | − |
| L. seeligeri | | + | + | − | − | − |
| L. grayi | | + | − | + | + | − |
| L. murrayi | | + | − | + | + | + |

- : 0 % de réaction positive

+ : 100 % de réaction positive

Ce tableau montre que les sept espèces composant le genre <u>Listeria</u> sont toutes séparées deux à deux par au moins l'un des cinq milieux sélectionnés.

Cette combinaison de milieu permet d'identifier simplement et précisément, toute souche de <u>Listeria</u>, quelle que soit l'espèce à laquelle elle appartient.

**Revendications**

**Revendications pour les Etats contractants suivants : BE, CH, LI, DE, GB, IT, LU, NL, PT**

1.  Procédé d'analyse bactériologique, selon lequel on met l'échantillon à analyser au contact d'un milieu d'identification comprenant un substrat chromogène ou fluorigène, susceptible d'être hydrolysé en présence d'au moins un enzyme associé au métabolisme d'au moins une espèce bactérienne, <u>caractérisé en ce que</u>, afin de différencier l'espèce <u>monocytogenes</u> dans un échantillon pouvant contenir des bactéries du genre <u>Listeria</u>, on introduit dans le milieu d'identification un substrat de la glycine aminopeptidase.

2.  Procédé selon la revendication 1, caractérisé en ce que la concentration du substrat de la glycine aminopeptidase varie entre 0,01 mM et 10 mM.

3.  Procédé selon la revendication 2, caractérisé en ce qu'on introduit un système tampon avec le substrat pour maintenir le pH à une valeur comprise entre 6 et 9.

4.  Procédé selon l'une quelconque des revendications précédentes, caractérisé en ce qu'on introduit dans le milieu d'identification au moins un activateur de la glycine aminopeptidase.

5.  Procédé selon la revendication 4, caractérisé en ce que l'activateur est un cation bivalent choisi dans le groupe consistant en $Mn^{++}$, $Mg^{++}$ et $Ca^{++}$.

6.  Milieu d'identification bactériologique, comprenant un substrat chromogène ou fluorigène, susceptible d'être hydrolysé en présence d'au moins un enzyme associé au métabolisme d'au moins une espèce bactérienne, caractérisé en ce que, afin de différencier l'espèce <u>monocytogenes</u> dans un échantillon à analyser mis au contact dudit milieu et pouvant contenir des bactéries du genre <u>Listeria</u>, le milieu d'identification comprend un substrat de la glycine aminopeptidase.

7.  Milieu selon la revendication 6, caractérisé en ce que le substrat est fluorigène directement, et est choisi parmi les composés suivants : glycine-7-amido-4-méthylcoumarine, glycine-7-amido-4-trifluorométhylcoumarine.

**8.** Milieu selon la revendication 6, caractérisé en ce que le substrat est chromogène directement et est notamment le glycine-4-nitroanilide.

**9.** Milieu selon la revendication 6, caractérisé en ce que le substrat est chromogène indirectement par addition d'un révélateur, et est choisi parmi les composés suivants : glycine-2-naphtylamide, glycine-4-méthoxy-2-naphtylamide.

**10.** Milieu selon la revendication 9, caractérisé en ce que le révélateur est un sel de diazonium tel que le "Fast Blue BB" ou le para-diméthylaminocinnamaldéhyde.

**11.** Milieu selon l'une quelconque des revendications 6 à 10, caractérisé en ce qu'il comprend un système tampon pour maintenir son pH entre 6 et 9.

**12.** Milieu selon l'une quelconque des revendications 6 à 11, caractérisé en ce que la concentration molaire du substrat est comprise entre 0,01 mM et 10 mM.

**13.** Milieu selon l'une quelconque des revendications précédentes, caractérisé en ce qu'il comprend en outre du chlorure de sodium, au moins un activateur de la glycine aminopeptidase.

**14.** Milieu selon la revendication 13, caractérisé en ce que l'activateur est un cation bivalent choisi dans le groupe consistant en $Mn^{++}$, $Mg^{++}$ et $Ca^{++}$.

**15.** Milieu selon la revendication 14, caractérisé en ce que la quantité de l'activateur est comprise entre 0 et 100 mg pour 1 litre.

**16.** Milieu selon l'une quelconque des revendications précédentes, caractérisé en ce qu'il est déshydraté.

**17.** Milieu selon la revendication 6, caractérisé en ce qu'il comprend en outre au moins un substrat de fermentation et/ou un substrat réductible et/ou un substrat enzymatique, dont la transformation chimique permet de caractériser l'espèce _Listeria_ présente dans l'échantillon à analyser.

**18.** Milieu selon la revendication 17, caractérisé en ce que le substrat de fermentation est choisi dans le groupe consistant en D-xylose, ribose, $\alpha$ -méthyl-mannoside, rhamnose, tagatose, $\alpha$-méthyl-D-glucoside et mannitol.

**19.** Milieu selon la revendication 17, caractérisé en ce que le substrat réductible est choisi parmi les nitrates, de préférence le nitrate de potassium.

**20.** Milieu selon la revendication 17, caractérisé en ce que le substrat enzymatique synthétique est un substrat de l'$\alpha$-mannosidase.

**21.** Dispositif d'identification des _Listeria monocytogenes_ comprenant un conditionnement d'un milieu selon l'une quelconque des revendications 6 à 20.

**Revendications pour l'Etat contractant suivant : ES**

**1.** Procédé d'analyse bactériologique, selon lequel on met l'échantillon à analyser au contact d'un milieu d'identification comprenant un substrat chromogène ou fluorigène, susceptible d'être hydrolysé en présence d'au moins un enzyme associé au métabolisme d'au moins une espèce bactérienne, caractérisé en ce que, afin de différencier l'espèce _monocytogenes_ dans un échantillon pouvant contenir des bactéries du genre _Listeria_, on introduit dans le milieu d'identification un substrat de la glycine aminopeptidase.

**2.** Procédé selon la revendication 1, caractérisé en ce que la concentration du substrat de la glycine aminopeptidase varie entre 0,01 mM et 10 mM.

**3.** Procédé selon la revendication 1, caractérisé en ce que le substrat est fluorigène directement, et est choisi parmi les composés suivants : glycine-7-amido-4-méthylcoumarine, glycine-7-amido-4-trifluorométhylcoumarine.

4. Procédé selon la revendication 1, caractérisé en ce que le substrat est chromogène directement, et est notamment le glycine-4-nitroanilide.

5. Procédé selon la revendication 1, caractérisé en ce que le substrat est chromogène indirectement par addition d'un révélateur, et est choisi parmi les composés suivants : glycine-2-naphtylamide, glycine-4-méthoxy-2-naphtylamide.

6. Procédé selon la revendication 5, caractérisé en ce que le révélateur est un sel de diazonium tel que le "Fast Blue BB" ou le para-diméthylaminocinnamaldéhyde.

7. Procédé selon la revendication 1, caractérisé en ce qu'on introduit un système tampon avec le substrat pour maintenir le pH à une valeur comprise entre 6 et 9.

8. Procédé selon l'une quelconque des revendications précédentes, caractérisé en ce qu'on introduit dans le milieu d'identification au moins un activateur de la glycine aminopeptidase.

9. Procédé selon la revendication 8, caractérisé en ce que l'activateur est un cation bivalent choisi dans le groupe consistant en $Mn^{++}$, $Mg^{++}$, $Ca^{++}$.

10. Procédé selon la revendication 9, caractérisé en ce que la quantité de l'activateur est comprise entre 0 et 100 mg pour 1 litre.

11. Procédé selon l'une quelconque des revendications précédentes, caractérisé en ce que le milieu est déshydraté.

12. Procédé selon la revendication 1, caractérisé en ce que le milieu comprend en outre au moins un substrat de fermentation et/ou un substrat réductible et/ou un substrat enzymatique, dont la transformation chimique permet de caractériser l'espèce Listeria présente dans l'échantillon à analyser.

13. Procédé selon la revendication 12, caractérisé en ce que le substrat de fermentation est choisi dans le groupe consistant en D-xylose, ribose, $\alpha$-méthyl-mannoside, rhammose, tagatose, $\alpha$-méthyl-D-glucoside et mannitol.

14. Procédé selon la revendication 12, caractérisé en ce que le substrat réductible est choisi parmi les nitrates, de préférence le nitrate de potassium.

15. Procédé selon la revendication 12, caractérisé en ce que le substrat enzymatique synthétique est un substrat de l'$\alpha$-mannosidase.

16. Dispositif d'identification des Listeria monocytogenes comprenant un conditionnement d'un milieu utilisé dans le procédé selon l'une quelconque des revendications 1 à 15.

**Patentansprüche**

**Patentansprüche für folgende Vertragsstaaten : BE, CH, LI, DE, GB, IT, LU, NL, PT**

1. Bakteriologisches Analyseverfahren, bei dem die zu analysierende Probe mit einem Identifikationsmilieu in Berührung gebracht wird, das ein chromogenes oder ein fluorigenes Substrat enthält, welches in der Gegenwart zumindest eines Enzyms, das mit dem Metabolismus zumindest einer Bakterienart in Verbindung steht, hydrolisierbar ist, dadurch gekennzeichnet, daß zur Differenzierung der monozytogenen Art in einer Probe, die Bakterien der Gattung Listeria enthalten kann, in das Identifikationsmilieu ein Glycinaminopeptidasesubstrat eingebracht wird.

2. Verfahren nach Anspruch 1, dadurch gekennzeichnet, daß die Konzentration des Glycinaminopeptida-

EP 0 496 680 B1

sesubstrates zwischen 0,01 mM und 10 mM variiert.

3. Verfahren nach Anspruch 2, dadurch gekennzeichnet, daß mit dem Substrat ein Puffersystem eingebracht wird, um den pH auf einem Wert zwischen 6 und 9 zu halten.

4. Verfahren nach einem der vorhergehenden Ansprüche, dadurch gekennzeichnet, daß in das Identifikationsmilieu zumindest ein Aktivator der Glycinaminopeptidase eingebracht wird.

5. Verfahren nach Anspruch 4, dadurch gekennzeichnet, daß der Aktivator ein zweiwertiges Kation, ausgewählt aus der Gruppe bestehend aus $Mn^{2+}$, $Mg^{2+}$ und $Ca^{2+}$, ist.

6. Bakteriologisches Identifikationsmilieu, enthaltend ein chromogenes oder ein fluorigenes Substrat, welches in der Gegenwart zumindest eines Enzyms, das mit dem Metabolismus zumindest einer Baktierenart in Verbindung steht, hydrolisierbar ist, dadurch gekennzeichnet, daß zur Differenzierung der monozytogenen Art in einer zu analysierenden Probe, die in Kontakt mit dem Milieu gebracht wurde, und die Bakterien der Gattung Listeria enthalten kann, das Identifikationsmilieu ein Glycinaminopeptidasesubstrat enthält.

7. Milieu nach Anspruch 6, dadurch gekennzeichnet, daß das Substrat direkt fluorigen ist, und daß es aus den folgenden Verbindungen ausgewählt ist, nämlich aus Glycin-7-amido-4-methylcumarin, Glycin-7-amido-4-trifluormethylcumarin.

8. Milieu nach Anspruch 6, dadurch gekennzeichnet, daß das Substrat direkt chromogen ist, und daß es insbesondere Glycin-4-nitroanilid ist.

9. Milieu nach Anspruch 6, dadurch gekennzeichnet, daß das Substrat indirekt chromogen durch Hinzufügen eines Entwicklers ist, und daß es aus den folgenden Verbindungen ausgewählt ist, nämlich Glycin-2-naphthylamid, Glycin-4-methoxy-2-naphthylamid.

10. Milieu nach Anspruch 9, dadurch gekennzeichnet, daß der Entwickler ein Diazoniumsalz, wie "Fast Blue BB" oder p-Dimethylaminozimtaldehyd ist.

11. Milieu nach einem der Ansprüche 6 bis 10, dadurch gekennzeichnet, daß es ein Puffersystem enthält, damit sein pH zwischen 6 und 9 gehalten wird.

12. Milieu nach einem der Ansprüche 6 bis 11, dadurch gekennzeichnet, daß die molare Konzentration des Substrates zwischen 0,01 mM und 10 mM liegt.

13. Milieu nach einem der vorhergehenden Ansprüche, dadurch gekennzeichnet, daß es darüber hinaus Natriumchlorid sowie zumindest einen Aktivator der Glycinaminopeptidase enthält.

14. Milieu nach Anspruch 13, dadurch gekennzeichnet, daß der Aktivator ein zweiwertiges Kation, ausgewählt aus der Gruppe bestehend aus $Mn^{2+}$, $Mg^{2+}$ und $Ca^{2+}$ ist.

15. Milieu nach Anspruch 14, dadurch gekennzeichnet, daß die Menge des Aktivators zwischen 0 und 100 mg pro Liter beträgt.

16. Milieu nach einem der vorhergehenden Ansprüche, dadurch gekennzeichnet, daß es dehydratisiert ist.

17. Milieu nach Anspruch 6, dadurch gekennzeichnet, daß es darüber hinaus zumindest ein Fermentationssubstrat und/oder ein reduzierbares Substrat und/oder ein enzymatisches Substrat enthält, deren chemische Umwandlung es ermöglicht, die in der zu analysierenden Probe vorhandene Art Listeria zu charakterisieren.

18. Milieu nach Anspruch 17, dadurch gekennzeichnet, daß das Fermentationssubstrat ausgewählt ist aus der Gruppe bestehend aus D-Xylose, Ribose, $\alpha$-Methylmannosid, Rhamnose, Tagatose, $\alpha$-Methyl-D-glycosid und Mannitol.

8

**19.** Milieu nach Anspruch 17, dadurch gekennzeichnet, daß das reduzierbare Substrat aus den Nitraten, vorzugsweise Kaliumnitrat, ausgewählt ist.

**20.** Milieu nach Anspruch 17, dadurch gekennzeichnet, daß das synthetische enzymatische Substrat ein Substrat von $\alpha$-Mannosidase ist.

**21.** Vorrichtung zur Identifizierung von <u>Listeria monocytogenes,</u> enthaltend eine Herrichtung eines Milieus nach einem der Ansprüche 6 bis 20.

**Patentansprüche für folgenden Vertragsstaat : ES**

**1.** Bakteriologisches Analyseverfahren, bei dem die zu analysierende Probe mit einem Identifikationsmilieu in Berührung gebracht wird, das ein chromogenes oder ein fluorigenes Substrat enthält, welches in der Gegenwart zumindest eines Enzyms, das mit dem Metabolismus zumindest einer Bakterienart in Verbindung steht, hydrolisierbar ist, dadurch gekennzeichnet, daß zur Differenzierung der <u>monozytogenen</u> Art in einer Probe, die Bakterien der Gattung <u>Listeria</u> enthalten kann, in das Identifikationsmilieu ein Glycinaminopeptidasesubstrat eingebracht wird.

**2.** Verfahren nach Anspruch 1, dadurch gekennzeichnet, daß die Konzentration des Glycinaminopeptidasesubstrates zwischen 0,01 mM und 10 mM variiert.

**3.** Verfahren nach Anspruch 1, dadurch gekennzeichnet, daß das Substrat direkt fluorigen ist, und daß es aus den folgenden Verbindungen ausgewählt ist, nämlich Glycin-6-amido-4-methylcumarin, Glycin-7-amido-4-trifluormethylcumarin.

**4.** Verfahren nach Anspruch 1, dadurch gekennzeichnet, daß das Substrat direkt chromogen ist, und daß es insbesondere Glycin-4-nitroanilid ist.

**5.** Verfahren nach Anspruch 1, dadurch gekennzeichnet, daß das Substrat indirekt chromogen durch Hinzufügen eines Entwicklers ist, und daß es aus den folgenden Verbindungen ausgewählt ist, nämlich Glycin-2-naphthylamid, Glycin-4-methoxy-2-naphthylamid.

**6.** Verfahren nach Anspruch 5, dadurch gekennzeichnet, daß der Entwickler ein Diazoniumsalz, wie "Fast Blue BB" oder p-Dimethylaminozimtaldehyd ist.

**7.** Verfahren nach Anspruch 1, dadurch gekennzeichnet, daß mit dem Substrat ein Puffersystem eingebracht wird, um den pH auf einem Wert zwischen 6 und 9 zu halten.

**8.** Verfahren nach einem der vorangehenden Ansprüche, dadurch gekennzeichnet, daß in das Identifikationsmilieu zumindest ein Aktivator für die Glycinaminopeptidase eingebracht wird.

**9.** Verfahren nach Anspruch 8, dadurch gekennzeichnet, daß der Aktivator ein zweiwertiges Kation, ausgewählt aus der Gruppe bestehend aus $Mn^{2+}$, $Mg^{2+}$, $Ca^{2+}$ ist.

**10.** Verfahren nach Anspruch 9, dadurch gekennzeichnet, daß die Menge des Aktivators zwischen 0 und 100 mg pro Liter liegt.

**11.** Verfahren nach einem der vorhergehenden Ansprüche, dadurch gekennzeichnet, daß das Milieu dehydratisiert ist.

**12.** Verfahren nach Anspruch 1, dadurch gekennzeichnet, daß das Milieu darüber hinaus zumindest ein Fermentationssubstrat und/oder ein reduzierbares Substrat und/oder ein enzymatisches Substrat enthält, deren chemische Umwandlung es ermöglicht, die in der zu analysierenden Probe vorhandene Art <u>Listeria</u> zu charakterisieren.

**13.** Verfahren nach Anspruch 12, dadurch gekennzeichnet, daß das Fermentationssubstrat ausgewählt ist aus der Gruppe bestehend aus D-xylose, Ribose, $\alpha$-Methylmannosid, Rhamnose, Tagatose, $\alpha$-Methyl-D-glucosid und Mannitol.

14. Verfahren nach Anspruch 12, dadurch gekennzeichnet, daß das reduzierbare Substrat ausgewählt aus den Nitraten, vorzugsweise Kaliumnitrat ist.

15. Verfahren nach Anspruch 12, dadurch gekennzeichnet, daß das synthetische enzymatische Substrat ein Substrat der α-Mannosidase ist.

16. Vorrichtung zur Identifizierung der <u>Listeria monocytogenes</u>, enthaltend eine Herrichtung eines Milieus wie es in dem Verfahren nach einem der Ansprüche 1 bis 15 verwendet wird.

**Claims**

**Claims for the following Contracting States : BE, CH, LI, DE, GB, IT, LU, NL, PT**

1. Process of bacteriological analysis according to which the sample to be analysed is brought into contact with an identification medium comprising a chromogenic or fluorigenic substrate capable of being hydrolysed in the presence of at least one enzyme associated with the metabolism of at least one bacterial species, characterised in that, in order to differentiate the <u>monocytogenes</u> species in a sample which may contain bacteria of the <u>listeria</u> genus, a glycine aminopeptidase substrate is introduced into the identification medium.

2. Process according to Claim 1, characterised in that the concentration of the glycine aminopeptidase substrate ranges between 0.01 mM and 10 mN.

3. Process according to Claim 2, characterised in that a buffer system is introduced with the substrate in order to maintain the pH to a value between 6 and 9.

4. Process according to any one of the preceding claims, characterised in that at least one glycine aminopeptidase activator is introduced into the identification medium.

5. Process according to Claim 4, characterised in that the activator is a bivalent cation chosen from the group consisting of $Mn^{++}$, $Mg^{++}$ and $Ca^{++}$.

6. Medium for bacteriological identification, comprising a chromogenic or fluorigenic substrate capable of being hydrolysed in the presence of at least one enzyme associated with the metabolism of at least one bacterial species, characterised in that in order to differentiate the <u>monocytogenes</u> species in a sample to be analysed brought into contact with the said medium and which may contain bacteria of the <u>listeria</u> genus the identification medium comprises a glycine aminopeptidase substrate.

7. Medium according to Claim 6, characterised in that the substrate is directly fluorigenic, and is chosen from the following compounds: glycine-7-amido-4-methylcoumarin, glycine-7-amido-4-trifluoromethylcoumarin.

8. Medium according to Claim 6, characterised in that the substrate is directly chromogenic and is in particular glycine-4-nitroanilide.

9. Medium according to Claim 6, characterised in that the substrate is indirectly chromogenic by adding an indicator, and is chosen from the following compounds: glycine-2-naphthylamide, glycine-4-methoxy-2-naphthylamide.

10. Medium according to Claim 9, characterised in that the indicator is a diazonium salt such as "Fast Blue BB" or para-dimethylaminocinnamaldehyde.

11. Medium according to any one of Claims 6 to 10, characterised in that it comprises a buffer system for maintaining its pH between 6 and 9.

12. Medium according to any one of Claims 6 to 11, characterised in that the molar concentration of the substrate is between 0.01 mM and 10 mM.

13. Medium according to any one of the preceding claims, characterised in that it comprises in addition to sodium chloride, at least one glycine aminopeptidase activator.

14. Medium according to Claim 13, characterised in that the activator is a bivalent cation chosen from the group consisting of $Mn^{++}$, $Mg^{++}$ and $Ca^{++}$.

15. Medium according to Claim 14, characterised in that the amount of activator is between 0 and 100 mg per 1 litre.

16. Medium according to any one of the preceding claims, characterised in that it is dehydrated.

17. Medium according to Claim 6, characterised in that in addition it comprises at least one fermentation substrate and/or one reducible substrate and/or one enzyme substrate, whose chemical conversion makes it possible to characterise the listeria species present in the sample to be analysed.

18. Medium according to Claim 17, characterised in that the fermentation substrate is chosen from the group consisting of D-xylose, ribose, $\alpha$-methyl-mannoside, rhamnose, tagatose, $\alpha$-methyl-D-glucoside and mannitol.

19. Medium according to Claim 17, characterised in that the reducible substrate is chosen from nitrates, preferably potassium nitrate.

20. Medium according to Claim 17, characterised in that the synthetic enzyme substrate is an $\alpha$-mannosidase substrate.

21. Device for identifying Listeria monocytogenes comprising a packaging of a medium according to any one of Claims 6 to 20.

**Claims for the following Contracting State : ES**

1. Process of bacteriological analysis according to which the sample to be analysed is brought into contact with an identification medium comprising a chromogenic or fluorigenic substrate capable of being hydrolysed in the presence of at least one enzyme associated with the metabolism of at least one bacterial species, characterised in that, in order to differenciate the monocytogenes species in a sample which may contain bacteria of the Listeria genus, a glycine aminopeptidase substrate is introduced into the identification medium.

2. Process according to Claim 1, characterised in that the concentration of the glycine aminopeptidase substrate ranges between 0.01 mM and 10 mM.

3. Process according to Claim 2, characterised in that the substrate is directly fluorigenic, and is chosen from the following compounds: glycine-7-amido-4-methylcoumarin, glycine-7-amido-4-trifluoromethylcoumarin.

4. Process according to Claim 1, characterised in that the substrate is directly chromogenic and is in particular glycine-4-nitroanilide.

5. Process according to Claim 1, characterised in that the substrate is indirectly chromogenic by adding an indicator, and is chosen from the following compounds: glycine-2-naphtlylamide, glycine-4-methoxy-2-naphtylamide.

6. Process according to Claim 5, characterised in that the indicator is a diazonium salt such as "Fast Blue BB" or para-dimethylaminocinnamaldehyde.

7. Process according to Claim 1, characterised in that a buffer system is introduced with the substrate in order to maintain the pH to a value between 6 and 9.

8. Process according to any one of the preceding claims, characterised in that at least one glycine aminopeptidase activator is introduced into the identification medium.

9. Process according to Claim 8, characterised in that the activator is a bivalent cation chosen from the group consisting of $Mn^{++}$, $Mg^{++}$, $Ca^{++}$.

10. Process according to Claim 9, characterised in that the amount of activator is between 0 and 100 mg per 1 litre.

11. Process according to any one of the preceding claims, characterised in that it is dehydrated.

12. Process according to Claim 1, characterised in that in addition it comprises at least one fermentation substrate and/or one reducible substrate and/or one enzyme substrate, whose chemical conversion makes it possible to characterise the Listeria species present in the sample to be analysed.

13. Process according to Claim 12, characterised in that the fermentation substrate is chosen from the group consisting of D-xylose, ribose, α-methyl-mannoside, rhamnose, tagatose, α-methyl-D-glucoside and mannitol.

14. Process according to Claim 12, characterised in that the reducible substrate is chosen from nitrates, preferably potassium nitrate.

15. Process according to Claim 12, characterised in that the synthetic enzyme substrate is an α-mannosidase substrate.

16. Device for identifying Listeria monocytogenes comprising a packaging of a medium used in a process according to any one of Claims 1 to 15.